# EUROPEAN PATENT SPECIFICATION

(11) **EP 1 179 176 B1**
(45) Date of publication and mention of the grant of the patent: **05.04.2006**
(21) Application number: 00923290.1
(22) Date of filing: 13.04.2000
(51) Int. Cl.: G01N 33/52, C07K 14/00, C12N 5/10, G01N 33/50

(54) **METHOD FOR THE DETECTION OF ANTIGEN SPECIFIC T-CELLS**
VERFAHREN ZUR DETEKTION VON ANTIGENSPEZIFISCHEN T-ZELLEN
PROCEDE DE DETECTION DE LYMPHOCYTES T SPECIFIQUES D' ANTIGENES

(30) Priority: 16.04.1999 US 129805 P
(43) Date of publication of application: 13.02.2002
(73) Proprietor: Ortho-McNeil Pharmaceutical, Inc., Raritan, NJ 08869-0606 (US)
(72) Inventor: CAI, Zeling, San Diego, CA 92130 (US); JACKSON, Michael, San Diego, CA 92121 (US); SEPULVEDA, Homero, San Diego, CA 92121 (US); HUANG, Jing-Feng, San Diego, CA 92130 (US)
(74) Representative: Mercer, Christopher Paul
(86) International application number: PCT/US2000/009851
(87) International publication number: WO 2000/063690

(56) References cited:
- WO-A-98/10284
- US-A- 5 284 935
- US-A- 5 529 921
- US-A- 5 583 031
- US-A- 5 595 881
- US-A- 5 734 023
- US-A- 5 820 866
- US-A- 5 869 270
- HUANG JING-FENG ET AL: "TCR-mediated internalization of peptide-MHC complexes acquired by T cells." SCIENCE (WASHINGTON D C), vol. 286, no. 5441, 29 October 1999 (1999-10-29), pages 952-954, XP002205112 ISSN: 0036-8075
- HWANG INKYU ET AL: "T cells can use either T cell receptor or CD28 receptors to absorb and internalize cell surface molecules derived from antigen-presenting cells." JOURNAL OF EXPERIMENTAL MEDICINE, vol. 191, no. 7, 3 April 2000 (2000-04-03), pages 1137-1148, XP002205113 ISSN: 0022-1007
- CLARK BRIAN B: "Fate of intercellular MHC-peptide-T-cell receptor complexes during T-cell activation." JOURNAL OF MOLECULAR RECOGNITION, vol. 8, no. 1-2, 1995, pages 63-66, XP001084326 10th International Symposium on Biorecognition and Affinity Technology;Gwatt-Thun, Switzerland; October 17-23, 1993 ISSN: 0952-3499
- MONKS COLIN R F ET AL: "Three-dimensional segregation of supramolecular activation clusters in T cells." NATURE (LONDON), vol. 395, no. 6697, pages 82-86, XP002205116 ISSN: 0028-0836
- CAI ZELING ET AL: "Transfected Drosophila cells as a probe for defining the minimal requirements for stimulating unprimed CD8+ T cells." PROCEEDINGS OF THE NATIONAL ACADEMY OF SCIENCES OF THE UNITED STATES, vol. 93, no. 25, 1996, pages 14736-14741, XP002205117 1996 ISSN: 0027-8424

## Description

### Background of the Invention

Activation of T-cells requires molecular interactions between TCR and MHC/peptide complexes on antigen-presenting cells (APCs). Although it is known that contact with these ligands is followed by TCR down regulation (1) and T-cell/APC interaction can cause APC-derived MHC molecules to adhere to the surface of T-cells (2, 3), the fate of MHC/peptide complexes on APCs is unclear. It has been hypothesized that recycling of MHC molecules allows a single MHC/peptide complex to trigger up to several hundred TCR molecules (4). Under such circumstances it is presumed that there is a transient association of MHC/peptide and TCR and the fate of MHC on APCs is not determined by TCR engagement. However, the formation of stable supramolecular activation clusters (SMACs) at the T-cell/APC interface (5) raises the question of how these complexes are dissociated.

### Summary of the Invention

The internalization of the MHC class I/antigen complexes by antigen specific T-cells has been utilized in the present invention to provide a method for the enrichment of antigen-specific T-cells from a heterogeneous population of T cells. The method of the present invention provides a means to purify individual antigen specific T cells, or to obtain a more homogeneous collection of T cells specific for a particular antigen from a mixture of T cells specific for a multitude of antigens. In addition, the method of the present invention provides a means to detect the presence of, and to quantify, T cells specific for a particular antigen present in a mixed population of T cells specific for a multitude of antigens.

### Brief Description of the Figures

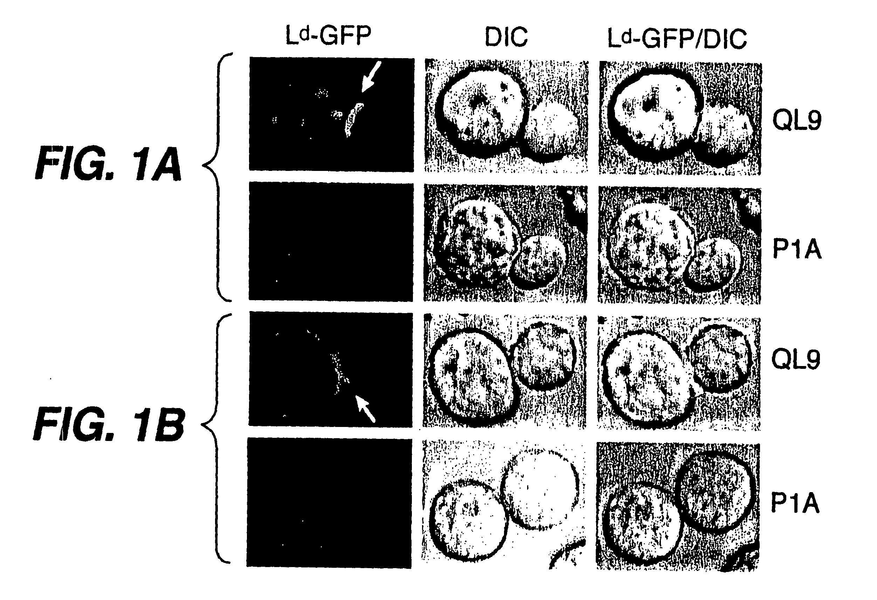

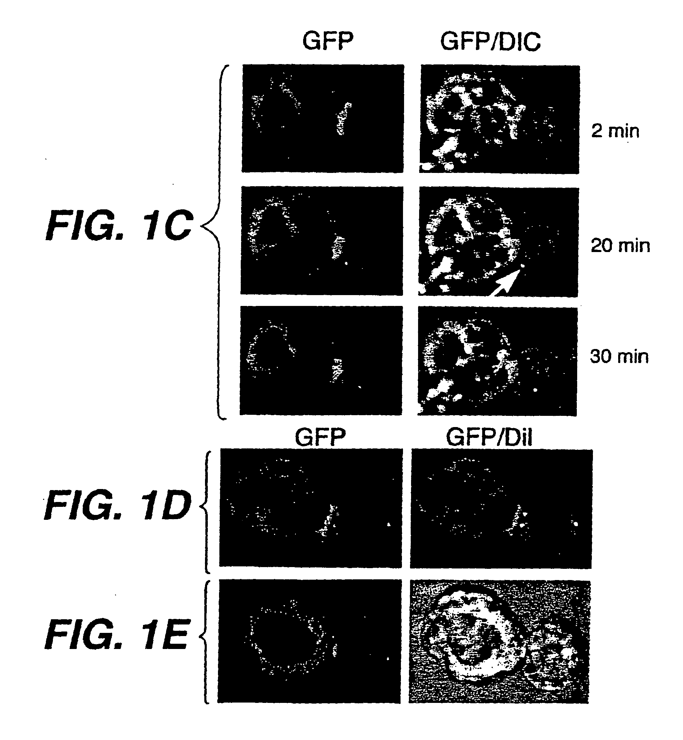

Figure 1. MHC class I molecules form clusters at T-cell/APC contact sites. Resting (A) or activated (B) CD8⁺ 2C T-cells were cultured with *Drosophila* APCs expressing L^{d-}GFP, B7-1 and ICAM-1 plus 10 ?M QL9 or P1A peptides at room temperature. GFP fluorescence was analyzed immediately after adding T-cells to APCs in a ΔTC3 culture dish (Bioptechs) using a confocal microscope system (Fluoview, Olympus). Left panels: L^{d}-GFP fluorescence. Middle panels: DIC (Differential Interference Contrast) images of T-cell/APC pairs. Right panels: overlay of L^{d}-GFP fluorescence and DIC images. (C) Resting CD8⁺ 2C T-cells acquiring L^{d}-GFP from *Drosophila* APCs (+QL9). Time-lapse imaging following L^{d-}GFP fluorescence in a T-cell/APC pair was carried out every 30 seconds. L^{d}-GFP fluorescence images taken at 5, 20 and 30 minutes respectively are shown in the left panels and the overlay images of L^{d}-GFP/ DIC are shown in the right panels. (D) Activated CD8⁺ 2C T-cell acquiring L^{d}-GFP from a *Drosophila* APC. Activated CD8⁺ 2C T-cells were pre-stained with 5 µM DiI (red) (Molecular Probes) before incubation with *Drosophila* APCs (+QL9). The images of a representative T-cell/APC pair are shown. (E) Acquisition of L^{d-}GFP from RMA.S cells (+QL9) by activated CD8⁺ 2C T-cells.

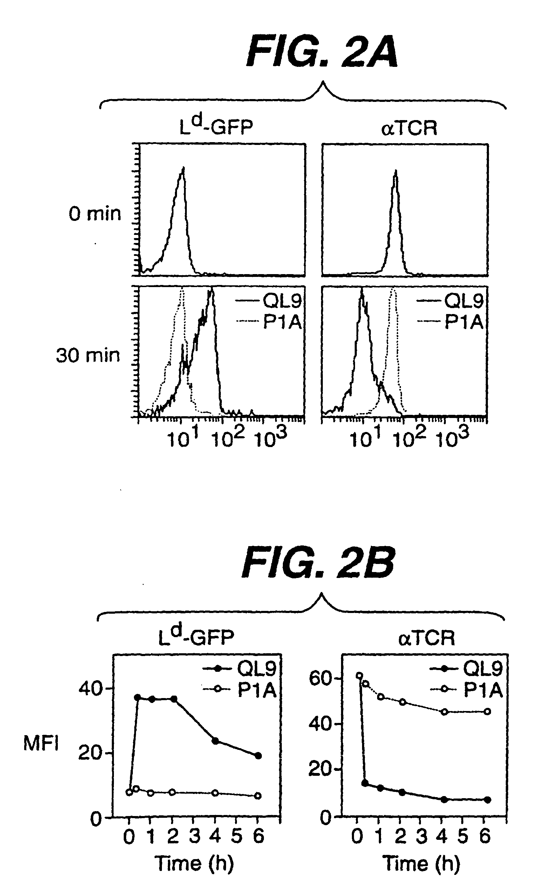

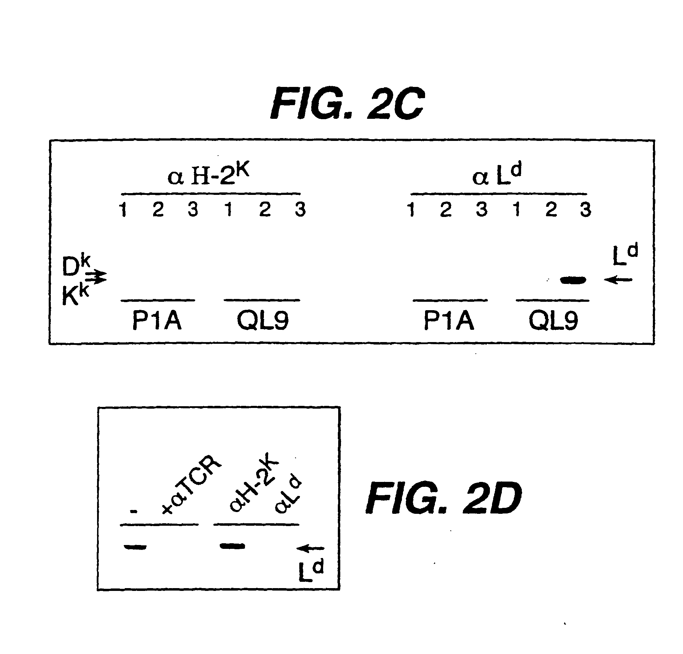

Figure 2. TCR-mediated acquisition of APC-derived MHC class I molecules by CD8⁺2C T-cells. Resting 2C T-cells were cultured with *Drosophila* cells expressing L^{d}-GFP, B7-1 and ICAM-1 loaded with QL9 or P1A peptide at 37°C for the indicated time. The total amount of L^{d}-GFP and surface level of TCR on CD8⁺ 2C T-cells was analyzed by FACS. (A) Expression of L^{d}-GFP and TCR on 2C T-cells at 0 and 30 minutes of culture. (B) Kinetics of L^{d}-GFP and TCR expression on 2C T-cells. The mean fluorescence intensity (MFI) of L^{d-}GFP and TCR expression on 2C T cells was analyzed at the indicated times with FACS. (C) Immunoprecipitation of APC-derived L^{d} class I molecules from 2C T-cells. A titrated number of 2C cells (lane 1: no T-cells, lane 2: 2 x 10⁷ and lane 3: 4 x10⁷) were cultured with 3 x 10^{6 35}S-methionine-labeled L cells expressing L^{d} (12). After culture for 4 hours, the 2C T-cells were purified from L cells (L-L^{d}) and the cell lysate of the purified 2C cells was immunoprecipitated with an anti-H-2^{k} mAb or an anti-L^{d}mAb (28-14-8) (PharMingen), respectively. (D) Acquisition of L^{d} molecules by T-cells is dependent on TCR/MHC/peptide interaction. 2C T-cells were cultured with L-L^{d} cells plus QL9 peptide for 4 hours in the presence or absence of mAbs as indicated. An anti-TCR mAb, 1B2 which recognizes both chains of the 2C TCR was used and immunoprecipitation of L^{d} was performed as described above.

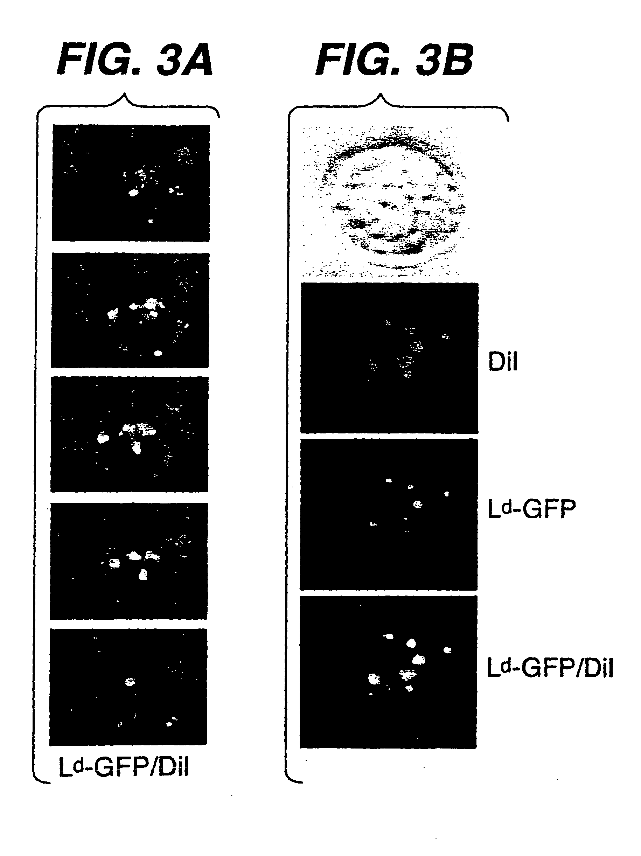

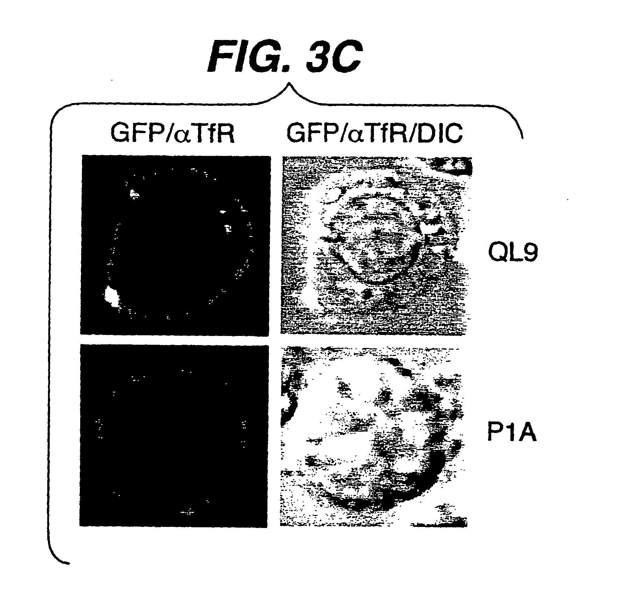

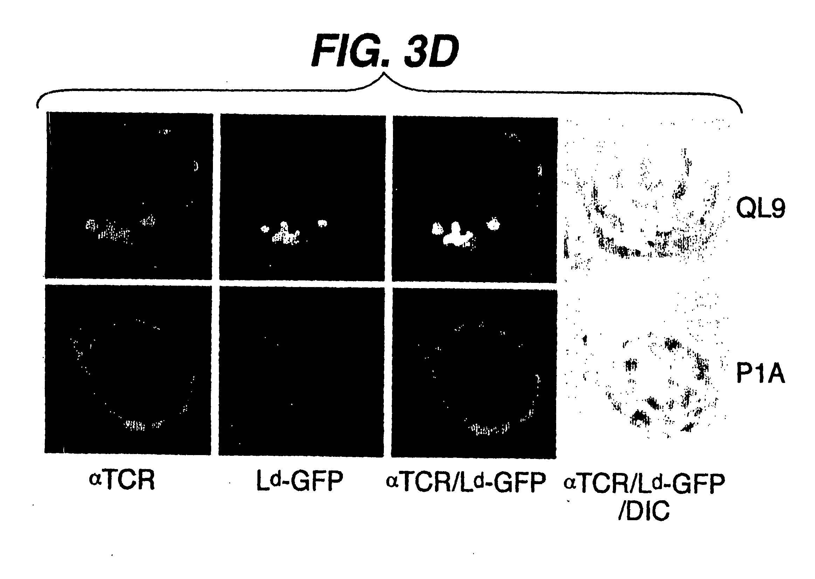

Figure 3. Internalization of APC-derived MHC class I molecules by T-cells. (A) Serial confocal images along the Z-axis of an activated 2C T-cell interacting with *Drosophila* APCs. CD8⁺2C T-cells were labeled with 5 ?M DiI (red) and cultured with *Drosophila* APCs expressing L^{d}-GFP (green) plus QL9 peptides for 30 min. (B) Co-localization of L^{d}-GFP with DiI-labeled membrane vesicles. 2C T-cells were incubated with QL9-loaded RMA.S cells expressing L^{d}-GFP at 37°C for 2 hours. (C) L^{d}-GFP acquired by 2C T-cells is in the cytoplasm. Activated CD8⁺ 2C T-cells were pretreated with lysosomal protease inhibitors (100 ?M Chloroquine and 50 ?M E64) and cultured with *Drosophila* APCs expressing L^{d-}GFP plus the indicated peptides for 1 hour. They were then stained with biotinylated antibody specific for transferrin receptor, followed by Strepavidin-Cyt3 (PharMingen). (D) Intracellular co-localization of TCR and L^{d}-GFP in 2C T-cells. After being cultured with *Drosophila* APC expressing L^{d}-GFP plus QL9 or P1A peptide for I hour, 2C cells were intracellularly stained with a cocktail of biotinylated mAb for TCR (anti-CD3?, anti-TCR? and a clonaltypic mAb,1B2) and subsequently with Strepavidin-Texas Red.

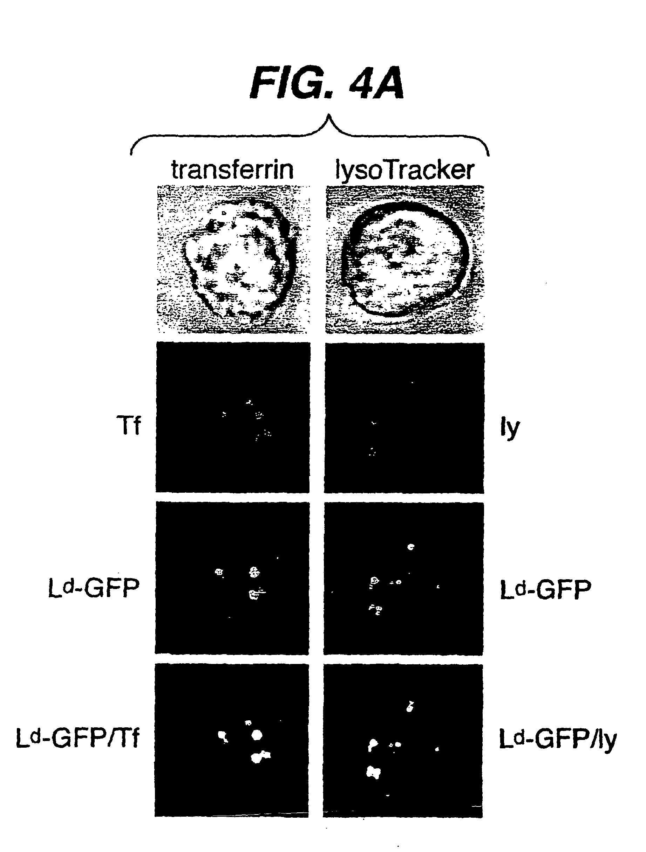

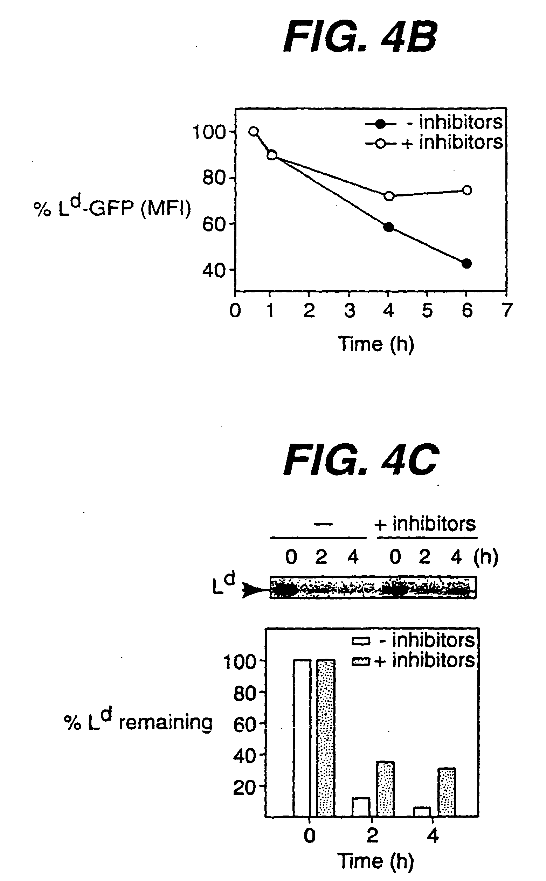

Figure 4. Endocytosis and degradation of APC-derived MHC class I molecules by T-cells. (A) Co-localization of L^{d}-GFP with transferrin (labeled as tf) and lysoTracker (labeled as ly) in 2C T-cells. Left panel images: activated CD8⁺ 2C T-cells were loaded with Texas red conjugated transferrin (5 ?g/ml) and incubated with QL9 peptide loaded *Drosophila* APCs (L^{d}-GFP) at 37°C for 1 hour. Right panel images: activated CD8⁺ 2C T-cells were incubated with QL9 loaded RMA.S cells expressing L^{d}-GFP, stained with 5 nM lysoTracker Red DND-99 (Molecular Probes). (B) Inhibition of L^{d}-GFP on 2C cells by lysosomal inhibitors. Resting CD8⁺ 2C T-cells were cultured with *Drosophila* APCs expressing L^{d}-GFP plus QL9 peptides in the presence or absence of a cocktail of lysosomal inhibitors (25 mM NH₄Cl, 10mM Chloroquine and 10 µM E64): After culture for the indicated time, L^{d}-GFP fluorescence intensity on CD8⁺ 2C cells was analyzed by FACS. (C) Degradation of APC-derived MHC class I molecules in 2C T-cells. 2C T-cells were cultured with ³⁵S-methionine labeled L^{d} transfected L cells for the indicated times in the absence or presence of NH₄Cl and E64. Immunoprecipitation of L^{d} was performed as described in Fig. 2. The amount of L^{d} remaining was quantified by densitometry.

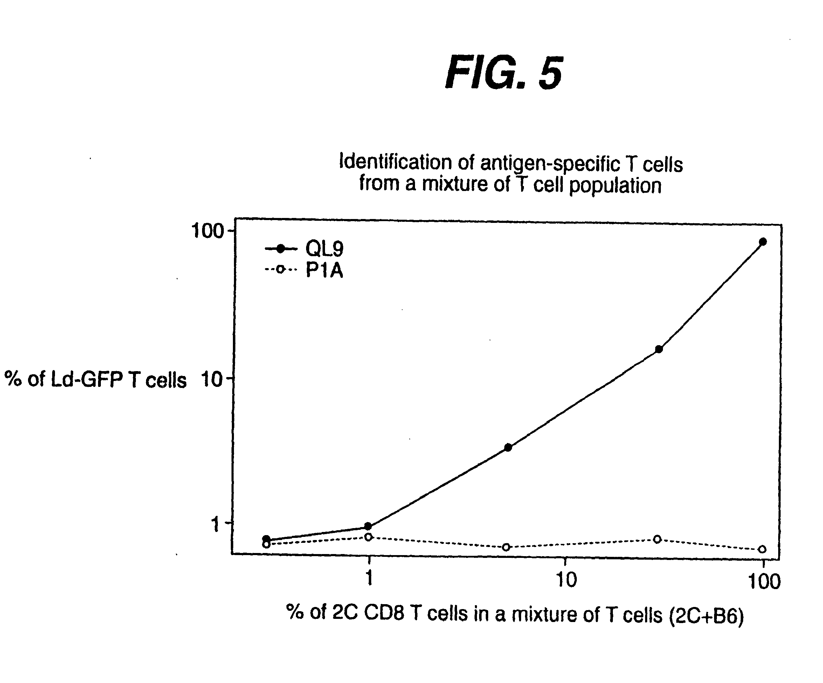

Figure 5, The separation by FACS of CD8+ 2C T-cells that have specifically taken up the GFP labeled MHC class I molecules loaded with antigen QL9 is shown using various ratios of 2C T-cells mixed with non-specific T-cells.

### Detailed Description the Invention

T-cell responses are initiated via contact with MHC class I/peptide complexes on antigen presenting cells (APCs). The fate of these complexes, however, is unknown. Here, using live APCs expressing MHC class I molecules fused with green-fluorescent protein, we show that peptide-specific T-cell/APC interaction induces clusters of MHC class I molecules to congregate within minutes at the contact site; thereafter, these MHC class I clusters are acquired by T-cells in small aggregates. We further demonstrate that acquisition of MHC class I by T-cells correlates with TCR down regulation, and the APC-derived MHC class I molecules are endocytosed and degraded by T-cells. These data also reveal a novel mechanism by which TCR recognition of MHC/peptide complexes can be curtailed by internalization of MHC molecules by T-cells.

To investigate the fate of MHC/peptide complexes on APCs after engagement of T-cells, we have generated stable mammalian and *Drosophila* cell lines that express MHC class I L^{d}-green fluorescent protein fusion molecules (L^{d}-GFP). A *Drosophila* cell expression vector containing L^{d}-GFP (JH 102) was constructed as follows: *Xho I* and *Sal I* cloning sites were generated before and after the stop codon of L^{d} in vector MJ262 (22) respectively by PCR mutagenesis. Then the DNA fragment of EGFP (*Xho I*/*Not 1*) was isolated from vector pEGFP-N3 (Clontech) and subcloned into the 3' end of L^{d} in the mutated MJ262 vector. The sequence of the new construct (JH102) was verified by DNA sequencing. It contains the full length sequence of L^{d} and EGFP. A linker sequence encoding 22 amino acids, which was derived from the multiple cloning sites of the vectors, was generated between the sequences of L^{d} and EGFP. Stable *Drosophila* cell lines expressing L^{d}-GFP with or without B7-1 and ICAM-1 molecules were generated as previously described (9). Construction of the L^{d}-GFP mammalian cell expression vector was as follows, the *Bam HI* DNA fragment containing L^{d} was isolated from vector JH102 and subcloned into vector pEGFP-N3 (Clontech). The resulting plasmid (JH103) was transfected into RMA.S cells by electroporation, and a stable cell line expressing L^{d}-GFP was generated by selection with G418 (1mg/ml). It is readily apparent to those of ordinary skill in the art that any means for the production of antigen associated-MHC class I molecules is suitable for use in the present invention. Examples of methods known in the art include, but are not limited to, those described in US patent 5,595,881, US patent 5,827,737 and US patent 5,731,160. It is also readily apparent to those of ordinary skill in the art that a variety of detectable markers, other than green fluorescent protein, can be fused to the MHC class I molecule, and are suitable for use in the methods of the present invention and can be linked to the MHC class I molecule by a wide variety of means. Examples of detectable markers that can be used in the method of the present invention include, but are not limited to, radioisotopes incorporated into or attached to the MHC class I protein, or any colorimetric or fluorescent compound or protein that can be linked to the MHC class I protein, for example by creating a recombinant fusion protein, by chemically linking the compounds or proteins post translationally, or by utilizing any binding pair partners such as antigen-antibody or streptavidin-biotin or avidin-biotin binding pairs to link the detectable marker to the protein.

L^{d}-GFP expressing cell lines were used as antigen presenting cells (APCs) to present specific QL9 peptide (7) to CD8⁺ T-cells from the 2C TCR transgenic-mouse line (2C T-cells), which specifically recognize the T-cell antigen QL9 (8). As previously reported for *Drosophila* cells expressing L^{d} (9), *Drosophila* cells expressing L^{d}-GFP plus two co-stimulating molecules, B7-1 and ICAM-1, induced peptide-specific TCR down regulation and strong proliferative responses of 2C cells, indicating that L^{d}-GFP molecules are functional. Unless stated otherwise, *Drosophila* cells co-transfected with L^{d}-GFP, B7-1 and ICAM-1 (L^{d}-GFP.B7.ICAM) were used as APCs. P1A peptide (10), which binds strongly to L^{d} but is not recognized by the 2C TCR (9), was used as a specificity control. It is readily apparent to one of ordinary skill in the art that any means for the presentation of antigen to the T cells is suitable for use in the methods of the present invention. A wide variety of antigen presenting systems are known, including but not limited to those described in US patent 5,595,881, US patent 5,827,737 and US patent 5,731,160. It is also readily apparent to those of ordinary skill in the art that ant T cell antigen is useful in the methods of the present invention. Any T cell antigen that can be associated with the MHC class I protein and presented to T cells is suitable for use in the present invention. Any source of such antigens is suitable for use in the present invention, whether the antigen is chemically synthesized or derived from a natural source. The antigens can be derived from any source and are not limited to any particular type, provided that the antigen can associate with MHC class I protein and present the antigen to the T cells.

Resting CD8⁺ 2C T-cells were purified and cultured with QL9-peptide-loaded *Drosophila* APCs for various periods; the dynamic interaction of T-cells and APCs was then investigated with a confocal microscope (FluoView, Olympus). Within a few minutes of interaction of 2C T-cells with APCs, L^{d}-GFP molecules formed large clusters at the site of T-cell contact (Fig.1A). In contrast, with addition of control P1A peptide, L^{d}-GFP remained homogeneously distributed on APCs after contact with T-cells (Fig.1A). In situations where a single T-cell bound to more than one APC, or one APC interacted with several T-cells, L^{d-}GFP clusters elicited by specific QL9 peptide were formed at each of the T-cell/APC contact sites. Similar results were obtained with pre-activated 2C T-cells (Fig. 1B). The formation of QL9-induced L^{d}-GFP clusters was not unique for *Drosophila* APCs since similar clusters occurred when L^{d}-GFP-transfected RMA.S cells (11), a mouse cell line, were used as APCs. Interestingly, QL9-dependent L^{d}-GFP cluster formation was also seen with *Drosophila* APCs transfected with L^{d}-GFP alone (without B7-1 or ICAM-1). Thus, the formation of MHC clusters at the T-cell/APC contact sites is mainly dependent on TCR/MHC/peptide interaction.

Time-lapse studies of T-cell/APC conjugates showed that the L^{d}-GFP clusters at the interface gradually decreased in size and eventually disappeared from the APC over a one hour period. Surprisingly, concomitant with the reduction in the size of L^{d}-GFP clusters, small punctuate aggregates of L^{d}-GFP appeared associated with the 2C T-cells as early as 15 minutes after engagement with APCs. Within 2 minutes of engagement of resting 2C T-cells with *Drosophila* APCs plus QL9 peptide (Fig. 1 C), a large cluster of L^{d}-GFP appeared at the contact site. However, after a further 20 minutes of culture, a small aggregate of L^{d}-GFP was apparent in the 2C T-cell; more L^{d}-GFP aggregates appeared in the 2C T-cell after 30 minutes of culture (Fig. 1C). The presence of L^{d}-GFP in T-cells was also investigated with pre-activated 2C T-cells (Fig. 1D,E). When activated 2C T-cells were cultured for 30 minutes with either L^{d}-GFP-transfected *Drosophila* APCs (Fig.1D) or RMA.S cells (Fig. 1E) plus QL9 peptides, multiple small aggregates of L^{d}-GFP were observed in the activated 2C T-cells. Aggregates of L^{d}-GFP also appeared in 2C T-cells activated by a lower affinity peptide, p2Ca (7), and no aggregates of L^{d}-GFP were seen in T-cells with the control P1A peptide. Thus, the acquisition of L^{d}-GFP appears to be peptide-specific.

The peptide-specific acquisition of L^{d}-GFP by T-cells was further studied by FACS analysis. As shown in Fig. 2A, L^{d}-GFP was detected on the majority of resting 2C T-cells after being cultured with *Drosophila* APCs plus QL9 peptide for 30 min. In contrast, L^{d}-GFP was not observed on 2C T-cells cultured with APCs loaded with control P1A peptide (Fig.2A). Kinetics studies showed that, with QL9 peptide, the amount of L^{d}-GFP acquired by 2C cells was maximal at 30 min and then gradually declined over several hours (Fig. 2B). By 16 hr, most of the L^{d}-GFP in T cells had disappeared. Acquisition of L^{d} by 2C T-cells was also confirmed by FACS analysis of L^{d} expression on 2C T-cells cultured with *Drosophila* APCs expressing L^{d} . Peptide titration studies showed that acquisition of L^{d}-GFP by T-cells was most prominent with a high concentration of QL9 peptide and was less marked, though significant, with a peptide of lower affinity for 2C cells, p2Ca peptide (7). The expression of co-stimulatory molecules (B7-1 and ICAM-1) by APCs did not enhance the acquisition of L^{d-}GFP molecules by 2C T-cells.

The results of an additional FACS analysis of T cells following incubation with APC and GFP labeled MHC is shown in Figure 5. A mixture ofT cells with indicated percentages of antigen-specific T cells (2C) and non-antigen specific T cells (B6) were cultured with Drosophila APC expressing MHC-GFP (L^{d}-GFP). After culturing with the antigenic peptide (QL9) or a control peptide (P1A) for 1 hour in 37°C, the L^{d}-GFP⁺ T cells were analyzed by FACS. The percentage of L^{d}-GFP⁺ T cells in each sample (Y axis) were plotted against the indicated percentage of antigen specific T cells (2C) in that sample (X axis). The data clearly demonstrate the separation of T cells that have taken up the GFP-label presented by the APC's, in the correct proportion to the amount of antigen specific T cells in the T cell mixture.

Uptake of APC-derived L^{d} molecules by 2C T-cells was further demonstrated by the acquisition of ³⁵S-labeled APC-derived MHC I molecules by T-cells in immuno-precipitation studies (Fig.2C). Fibroblasts (L cell) expressing L^{d} (12) were used as APCs for these studies since unlike *Drosophila* cells, they are adherent, a property that greatly aids in the isolation of a pure population of T-cells from the APC/T-cell mixture. After culturing 2C cells with L^{d}-transfected L cells (L-L^{d} plus QL9 peptide, L^{d} could be immuno-precipitated from 2C cells, reaching a peak at 4 hours of culture. The amount of L^{d} precipitated from 2C cells closely correlated with the numbers of 2C T-cells in the culture (Fig 2C). In the presence of a control peptide (P1A), however, precipitation of L^{d} was very limited. Other class I molecules expressed on L cells (D^{k} and K^{k}) were not detectable in 2C T-cells by immunoprecipitation (Fig. 2C). Importantly, the peptide-dependent acquisition of L^{d} molecules by T-cells could be blocked by adding either anti-TCR or anti-L^{d} mAbs to the culture, indicating that the acquisition of L^{d} by T-cells requires a specific interaction between TCR and MHC/peptide (Fig. 2D).

It is notable that rapid acquisition of L^{d} molecules by T-cells correlated with equally rapid down-regulation of 2C TCR (Fig.2B). Since TCR down-regulation reflects internalization by T-cells (13), L^{d} molecules might also be internalized. To address this question, a lipid-soluble fluorescence dye (DiI) (Molecular Probes) (14) was used to label the membrane of activated 2C cells. As shown in Fig. 3A, after culturing T-cells with APCs expressing L^{d}-GFP plus QL9 peptide for 30 min, substantial numbers of small L^{d}-GFP aggregates were detected in activated T-cells. Some of the L^{d}-GFP aggregates were clearly inside the T-cells while others remained at the T-cell/APC contact site (Fig.3A). Further culturing of the DiI-labeled 2C T-cells with APCs for 2 hours resulted in conspicuous aggregates of L^{d}-GFP inside 2C T-cells and these aggregates co-localized with DiI-labeled membrane vesicles (Fig.3B).

The intracellular localization of L^{d}-GFP in 2C T-cells was further confirmed by surface-staining of 2C T-cells with a monoclonal antibody specific for transferrin receptor (Fig.3C). When activated 2C T-cells were cultured with APCs expressing L^{d}-GFP for 1 hr in the presence of QL9 peptide, L^{d}-GFP aggregates were detected inside the T-cells and showed a perinuclear distribution (Fig.3C). In contrast, no L^{d}-GFP aggregates were observed in 2C T-cells when P1A peptide was used (Fig.3C).

The above observation that L^{d} molecules acquired by T-cells from APCs can be internalized raises the question of how this process occurs. Soluble ligands are known to be internalized through endocytosis via clathrin-coated pits (15). Because internalization of L^{d-}GFP by T-cells was dependent on interaction of TCR and MHC/peptide, and L^{d}-GFP co-localized with TCR (Fig. 3D), it is possible that MHC molecules from APCs are internalized through TCR-mediated endocytosis.

Since transferrin is internalized by cells through receptor-mediated endocytosis (13), we used transferrin conjugated to Texas Red as a marker to follow the intracellular fate of L^{d-}GFP in 2C T-cells. As shown in Fig. 4A, transferrin was internalized by T-cells and was associated with multiple membrane vesicles. The L^{d}-GFP aggregates internalized by T-cells displayed a similar pattern of intracellular distribution (Fig. 4A). The overlay images of transferrin and L^{d}-GFP indicated that the L^{d}-GFP aggregates internalized by T-cells co-localized with transferrin-containing vesicles (Fig.4A). These data strongly suggest that, after interaction with TCR, APC-derived MHC molecules are internalized by T-cells through endocytosis.

LysoTracker, a red fluorescent dye which specifically accumulates in low pH compartments of cells (16), was used as a marker for lysosomes to track the intracellular fate of L^{d}-GFP. As shown in Fig 4A, after culturing 2C T-cells with APCs for 1 hour, L^{d}-GFP appeared in the acidic compartments of T-cells, as indicated by lysoTracker dye. The presence of L^{d} in lysosomes was further confirmed by immune staining of fixed 2C T-cells with a mAb specific for LAMP-1, a lysosome associated membrane molecule.

The co-localization of L^{d}-GFP with lysoTracker and LAMP-1 suggests that L^{d}-GFP endocytosed by 2C T-cells was subjected to lysosomal degradation. To examine this possibility, 2C cells were cultured with L^{d}-GFP *Drosophila* APCs plus QL9 peptide in the presence or absence of lysosomal inhibitors (NH₄Cl, Chloroquine and E64) for up to 6 hours and then analyzed by FACS for total amount of L^{d}-GFP. As shown in Fig 4B, the disappearance of L^{d}-GFP in 2C cells was clearly inhibited by the addition of lysosomal inhibitors.

Similar findings were seen with the immunoprecipitation of L^{d} (Fig 4C). In the experiment shown, 2C cells were first cultured for 4 hours with ³⁵S-labeled L^{d}-transfected L cells plus QL9 peptide; to prevent further uptake of L^{d}, 2C cells were then separated from the APCs and cultured for 2-4 hr in the presence or absence of lysosomal inhibitors (NH₄Cl and E64). Under these APC-free conditions, L^{d} molecules in 2C cells disappeared more rapidly for cells cultured in medium alone than for cells cultured with the inhibitors.

Several studies have shown that T-cell/APC interaction can cause a number of molecules from APCs to adhere to the surface of T-cells (2, 3). The present disclosure demonstrates that, for CD8⁺ 2C cells, MHC class I molecules (L^{d}) on APCs are acquired by T-cells after forming supramolecular activation clusters (SMACs) at the site of T-cell/APC interaction (3); the appearance of APC-derived MHC class I molecules in SMACs is peptide-dependent and occurs rapidly. In addition, we show that after binding to TCR, APC-derived MHC class I molecules are endocytosed by T-cells and subsequently degraded through a lysosomal pathway. Interestingly, T-cells can also internalize B7 molecules from APCs (3). It is unclear whether B7 is degraded post internalization.

Antigen specific interaction of T-cells and APCs induces TCR internalization and degradation in lysosomes in an antigen dose- and time-dependent manner (17). Here, we demonstrated that the requirements and the kinetics for internalization and degradation of APC-derived MHC I molecules are similar to that for internalization and degradation of TCR and that APC-derived MHC co-localizes with TCR in T-cells. These findings strongly suggest that MHC I molecules and TCR are internalized and degraded together by T-cells. According to the serial triggering model for T-cell activation, transient association of MHC/peptide with TCR is required for consecutive triggering of multiple TCRs (1,4). However, our finding that after specific interaction with TCR, MHC molecules form stable clusters and subsequently are internalized with TCR suggests that the TCR/MHC/peptide interaction is not transient. This raises a question concerning the role of co-internalization of MHC and TCR in T-cell activation.

In the case of soluble ligands such as growth factors and hormones, internalization is known to be involved in signal transduction (18). Hence, internalization of MHC molecules by T-cells may contribute to TCR-mediated intracellular signal transduction (19) and co-localization of TCR and MHC in T-cells may be required for sustained TCR signaling (20). A similar, but unrelated, observation is provided by the finding that internalization of a seven-transmembrane ligand (boss) via a specific receptor on adjacent T-cells is important for eye development in insects (21).

An alternative possibility is that internalization of MHC molecules during T-cell/APC interaction is a device to protect the responding T-cells from excessive stimulation from APC. Here, it is notable that binding of MHC class I molecules to T-cells correlates closely with TCR down regulation: both processes have similar kinetics, are independent of co-stimulation molecules and are much less prominent with low concentrations of MHC-bound peptides. Hence, for T-cell interaction with APC expressing high concentrations of peptides, rapid internalization of TCR/MHC/peptide complexes may serve to reduce the intensity of TCR signaling and thus lessen the risk of tolerance induction.

### References

1. S. Valitutti, S. Mueller, M. Cella, E. Padovan, A. Lanzavecchia, *Nature (London)* 375, 148-50 (1995); Z. Cai, et al., *J. Exp. Med.* 185, 641-651 (1997). T. R Preckel, M.S. Grimm, H. U. Weltzier, J. *Exp. Med.* 185, 1803-1813 (1997).
2. M. K. Saizawa, S. Suzuko, *J. Cell Biochem.* 16D" 54 (1992).
3. M. I. Lorber, M. R.Loken, A. M. Stall, F. W. Fitch, *Journal of Immunology* 128, 2798-2803 (1982).
4. S. Valitutti, A. Lanzavecchia, *Immunologist* 3, 122-4 (1995); S. Valitutti, A. Lanzavecchia, *Immunol. Today* 18, 299-304 (1997).
5. C. R. F. Monks, B. A. Freiberg, H. Kupfer, N. Sciaky, A. Kupfer, *Nature (London)* 395, 82-86 (1998).
6. reserved
7. Y. Sykulev, et *al., Immunity* 1*,* 15-22 (1994).
8. W. C. Sha, et al., *Nature* 335, 271-274 (1988).
9. Z. Cai, et al., *Proc. Natl. Acad. Sci. U.S.A.* 93, 14736-14741 (1996).
10. B. van den Eynde, B. Lethe, A. van Pel, E. De Plaen, T. Boon, *J. Exp. Med.* 173, 1373-1384 (1991).
11. Townsend, et al. *Cell* 62, 285-95 (1990).
12. J. K. Pullen, H. D. Hunt, L. R. Pease, *J. Immunol.* 146, 2145-51 (1991).
13. S. Valitutti, S. Muller, M. Salio, A. Lanzavecchia, *J. Exp. Med.* 185, 1859-1864 (1997).
14. M.G. Honig, R.I. Hume, *J. Cell Biol* 103, 171-187 (1986).
15. S. Mukherjee, R. N. Ghosh, F. R Maxfield, *Physiol. Rev.* 77, 759-803 (1997).
16. R. Wubbolts, et al., *J*. *Cell Biol.* 135, 611-622 (1996).
17. S. Valitutti, S. Muller, M. Salio, A. Lanzavecchia, *J. Exp. Med.* 185, 1859-1864 (1997).
18. L. Xue, J. Lucocq, *Cell. Signalling* 10(5),339-348 (1998); B.P..Ceresa, et al., *Mol. Cell. Biol.* 18 (7), 3862-3870 (1998); J.C. Chow, G. Condorelli, R. J. Smith, *J. Biol. Chem.* 273(8), 4672-4680 (1998).
19. A. Weiss, D. R Littman, *Cell* 76, 263-274 (1994); C. R. F. Monks, H. Kupfer, I. Tamir, A. Barlow, A. Kupfer, *Nature (London)* 385, 83-86 (1997); R. N. Gernain, Current Biology 7, -R644A (1997); J. J. Boniface et al. *Immunity* 9, 459-466 (1998).
20. A. Lanzavecchia, *J. Exp. Med.* 185, 1717-1719 (1997).
21. H. Kramer, R. L. Cagan, S. L. Zipursky, *Nature (London)* 352, 207-12 (1991); R. L. Cagan, H. Kramer, A. C. Hart, L. S. Zipursky, *Cell* 69, 393-399 (1992); H. Kramer, M. Phistry, *J. Cell Biol.* 133, 1205-1215 (1996).
22. M. R. Jackson, E. S. Song, Y. Yang, P. A. Peterson, *Proc.Natl. Acad. Sci. USA 89,* 12117-12121 (1992).

## Claims

1. A method for the detection of antigen specific T cells, comprising:
a. providing a recombinant cell expressing an MHC class I protein-fluorescent protein fusion molecule or a radiolabeled MHC class I protein on a surface of the recombinant cell;
b. presenting an antigen associated with the MHC class I molecule of part (a) to a population of T cells;
c. incubating the fusion molecule or the radiolabeled MHC class I protein bound to the specific antigen together with the population of T cells, for a period of from 2 minutes to 6 hours, for the T cells to internalize the fusion molecule or the radiolabeled MHC class I protein from the T cell surface; and
d. identifying the T cells that acquired the detectable marker.

2. The method of claim 1, wherein said fluorescent protein is green fluorescent protein.

3. The method of claim 1, wherein the identifying of the T cells that have acquired the MHC class I protein-fluorescent protein fusion molecule is done by detecting fluorescent emission of the fluorescent protein fusion molecule.

4. The method of claim 1, wherein the identifying of the T cells that have internalized the MHC class I protein-fluorescent protein fusion molecule is done by detecting fluorescent emission of the fluorescent protein fusion molecule in a fluorescence activated cell sorter.

5. The method of claim 1, wherein said recombinant cell is a *Drosophila* cell.

## Revendications

1. Méthode pour la détection de cellules T spécifiques d'un antigène, comprenant:
a. prévoir une cellule recombinante exprimant une molécule de fusion protéine fluorescente-protéine de MHC classe I ou une protéine de MHC classe I radiomarquée sur une surface de la cellule recombinante;
b. présenter un antigène associé à la molécule de MHC classe I de la partie (a) à une population de cellules T;
c. soumettre à incubation la molécule de fusion ou la protéine de MHC classe I radiomarquée liée à l'antigène spécifique, avec la population des cellules T, pendant une période de 2 minutes à 6 heures, pour que les cellules T internalisent la molécule de fusion ou la protéine de MHC classe I radiomarquée de la surface des cellules T; et
d. identifier les cellules T qui ont acquis le marqueur détectable.

2. Méthode de la revendication 1, où ladite protéine fluorescente est une protéine fluorescente verte.

3. Méthode de la revendication 1, où l'identification des cellules T qui ont acquis la molécule de fusion protéine fluorescente-protéine de MHC classe I est faite en détectant l'émission fluorescente de la molécule de fusion de la protéine fluorescente.

4. Méthode de la revendication 1, où l'identification des cellules T qui ont internalisé la molécule de fusion protéine fluorescente-protéine de MHC classe I est faite en détectant l'émission fluorescente de la molécule de fusion de la protéine fluorescente dans un trieur de cellules activé par la fluorescence.

5. Méthode de la revendication 1, où ladite cellule recombinante est une cellule de *Drosophila.*

## Patentansprüche

1. Verfahren zum Nachweis von Antigen-spezifischen T-Zellen, umfassend:
a. zur Verfügung stellen einer rekombinanten Zelle, die ein MHC Klasse I Proteinfluoreszierendes Proteinfusionsmolekül oder ein radiomarkiertes MHC Klasse I Protein auf einer Oberfläche der rekombinanten Zelle exprimiert;
b. Präsentieren eines Antigens, das mit dem MHC Klasse I Molekül von Teil (a) assoziiert ist, einer Population von T-Zellen;
c. Inkubieren des Fusionsmoleküls oder des radiomarkierten MHC Klasse I Proteins, gebunden an das spezifische Antigen, zusammen mit der Population von T-Zellen, für eine Zeitdauer von 2 Minuten bis zu 6 Stunden, damit die T-Zellen das Fusionsmolekül oder das radiomarkierte MHC Klasse I Protein von der T-Zelloberfläche internalisieren; und
d. Identifizieren der T-Zellen, die den nachweisbaren Marker aufgenommen haben.

2. Verfahren nach Anspruch 1, wobei das fluoreszierende Protein grünes fluoreszierendes Protein ist.

3. Verfahren nach Anspruch 1, wobei das Identifizieren der T-Zellen, die das MHC Klasse I Protein-fluoreszierende Proteinfusionsmolekül aufgenommen haben, durch Nachweisen von Fluoreszenzemission des Fluoreszenz-Proteinfusionsmoleküls durchgeführt wird.

4. Verfahren nach Anspruch 1, wobei das Identifizieren der T-Zellen, die das MHC Klasse I Protein-fluoreszierende Proteinfusionsmolekül internalisiert haben durch Nachweisen von Fluoreszenzemission des Fluoreszenz-Proteinfusionsmoleküls in einem Fluoreszenzaktivierten Zell Sorter durchgeführt wird.

5. Verfahren nach Anspruch 1, wobei die rekombinante Zelle eine *Drosophila* Zelle ist.
